# EUROPÄISCHE PATENTANMELDUNG

(11) **EP 1 001 056 A1**
(43) Veröffentlichungstag der Anmeldung: **17.05.2000**
(21) Anmeldenummer: 99121540.1
(22) Anmeldetag: 29.10.1999
(51) Int. Cl.: D01F 1/10, D01F 6/04, D01F 6/06, D01F 6/62, D04H 1/42, C08K 5/20

(54) **Verfahren zur hydrophilen Ausrüstung von Fasern auf Basis von Polyolefinen oder Polyester unter Einsatz von Fettsäureamiden**

(30) Priorität: 10.11.1998 DE 19851683
(71) Anmelder: Cognis Deutschland GmbH, 40589 Düsseldorf (DE)
(72) Erfinder: Padurschel, Petra, 41515 Grevenbroich (DE); Birnbrich, Paul, Dr., 42719 Solingen (DE); Bialas, Norbert, Dr., 41539 Dormagen (DE); Mathis, Raymond, Dr., 40627 Düsseldorf (DE); Wucherpfenning, Sven, 40595 Düsseldorf (DE)

(57) **Zusammenfassung**

Gegenstand der Erfindung ist ein Verfahren zur hydrophilen Ausrüstung von Fasern, die ausschließlich oder ganz Polyolefine oder Polyester enthalten, wobei man eine Mischung enthaltend (a) überwiegend Polyolefine oder Polyester und (b) 0,01 bis 10 Gew.-% - bezogen auf die Summe von Polyolefin oder Polyester - einer Zusammensetzung, die mindestens eine Verbindung aus der Klasse der Fettsäureamide enthält, bei Temperaturen im Bereich von 180 bis 320 °C auf übliche Weise einer formgebenden Verarbeitung zu Fasern unterwirft, wobei man
(i) der Mischung, die man der formgebenden Verarbeitung unterwirft, vor oder während dieser formgebenden Verarbeitung keine Übergangsmetallverbindungen zudosiert,
(ii) die Fettsäureamide (b) auswählt aus der Klasse der Fettsäurealkanolamide oder - dialkanolamide, wobei die diesen Verbindungen zu Grunde liegenden Fettsäuren gesättigte Fettsäuren mit 12-22 C-Atomen sind und daß
(iii) die Fettsäureamide (b) im individuellen Benetzungstest mindestens vier Cyclen bis zum Versagen benötigen.

## Beschreibung

### Gebiet der Erfindung

Die Erfindung betrifft ein Verfahren zur hydrophilen Ausrüstung von Fasern, die ausschließlich oder ganz Polyolefine oder Polyester enthalten. Dabei kommen spezielle Fettsäureamide zum Einsatz.

### Stand der Technik

In zahlreichen Fällen muß die Oberfläche von Kunststoff-Erzeugnissen mit speziellen Effekten versehen werden, die sich während der Formgebung entweder aus technischen Gründen gar nicht bzw. nur unvollkommen oder aber aus wirtschaftlichen Gründen nur unvorteilhaft erzeugen lassen. Ein solcher Effekte ist beispielsweise die Verbesserung der Benetzbarkeit mit polaren Flüssigkeiten wie Wasser - technische Anwendungen liegen hier beispielsweise auf dem Gebiet der Herstellung von Hygieneartikeln.

Bei der Herstellung von Hygieneartikeln, wie Windeln oder Damenbinden, werden absorbierende Materialien verwendet, um wäßrige Flüssigkeiten aufzunehmen. Um den direkten Kontakt mit dem absorbierenden Material beim Tragen zu verhindern und den Tragekomfort zu erhöhen wird dieses Material mit einem dünnen, wasserdurchlässigen Vliesstoff umhüllt. Derartige Vliesstoffe werden üblicherweise aus synthetischen Fasern, wie Polyolefin- oder Polyesterfasern hergestellt, da diese Fasern preiswert zu produzieren sind, gute mechanische Eigenschaften aufweisen und thermisch belastbar sind. Allerdings eignen sich unbehandelte Polyolefin- oder Polyesterfasern für diesen Einsatzzweck nicht, da sie aufgrund ihrer hydrophoben Oberfläche keine ausreichende Durchlässigkeit für wäßrige Flüssigkeiten aufweisen.

Zu diesem Zweck muß die Faseroberfläche durch eine entsprechende Präparation hydrophil ausgerüstet werden. Gewünscht ist weiterhin, daß die hydrophile Ausrüstung der Faser möglichst lange erhalten bleibt, ohne daß die Wasserdurchlässigkeit des Vliesstoffs verringert wird. Werden derartige Vliesstoffe beispielsweise in Windeln verarbeitet, können diese mehrfach beansprucht werden, ohne wasserundurchlässig zu werden. Auf diese Weise wird die Tragezeit der Windeln erhöht und der durch verbrauchte Windeln verursachte Abfall verringert.

**US-A-5,045,387** beschreibt beispielsweise ein Mittel zur hydrophilen Ausrüstung von Polyolefinfasern welches eine Mischung aus einem alkoxyliertem Ricinolsäurederivat, einem hydrierten Ricinolsäurederivat, einer C₁₈-Fettsäure und einem polyalkoxylierten Polymethylsiloxan enthält. Dieses Mittel muß von außen auf die Oberfläche der Fasern oder Folien aufgebracht werden.

**US-A-5,654,086** beschreibt die hydrophile Ausrüstung von ansonsten hydrophoben Fasern auf Basis thermoplastischer Kunststoffe, wobei man die hydrophobe Faser mit einer Mischung aus fünf oberflächenaktiven Substanzen (Tensiden) behandelt. Dabei handelt es sich um (A) Polyoxyalkylen-Addukte von C₂₈₋₅₀-Alkoholen oder -Alkylaminen oder Fettsäureamiden auf Basis von C₃₀₋₅₀-Fettsäuren, (B) Polyoxyalkylen-Addukte von Fettsäureamiden auf Basis von C₂₀₋₂₈-Fettsäuren, (C) Fettsäureamide auf Basis von C₁₆₋₂₈-Fettsäuren und Alkanolaminen, (D) Polyoxyalkylen-Addukte C₁₀₋₂₂-Alkylphosphat-Salzen und (E) C₁₂₋₁₆-Alkylsulfonatsalzen.

**EP-A-400,622** beschreibt ein Verfahren, um nichtgewebten Materialien, die hydrophobe Fasern enthalten, hydrophile Eigenschaften zu verleihen. Dabei wird eine Zusammensetzung, die spezielle polyoxyalkylierte sekundäre oder tertiäre Amine enthält, vor dem Verspinnen in eine geschmolzene Zusammensetzung eingebracht, die Polyolefin enthält.

**EP-B-372 890** beschreibt Fasern auf Polyolefin- oder Polyester-Basis mit einem mit der Oberfläche verhafteten Schmiermittel. Dieses Schmiermittel umfaßt eine Mischung aus (1) Fettsäurediethanolamid, (2) einem Polyether-modifizierten Silikon, (3) einem Sorbitan-Fettsäureester und (4) einem Metallsalz eines Alkylsulfonats; dabei liegen die Komponenten (1) bis (4) in speziellen Mengenverhältnissen vor. Gemäß Seite 3, Zeilen 20-26 wird die Mischung der Komponenten (1) bis (4) auf die Oberfläche aufgebracht. Diese Technik des Aufbringens der die vier Komponenten enthaltenden Mischung auf die Oberfläche bereits fertiger Fasern wird auch auf Seite 4, Zeilen 6-9 nochmals näher erläutert. Dort sind als Aufbring-Techniken genannt: a) der Einsatz von Rollen, b) ein Aufsprühen und c) das Eintauchen. Es handelt sich demnach um ein Verfahren, bei dem eine Mischung der Komponenten (1) bis (4) in einem zusätzlichen Verarbeitungsschritt auf die Oberfläche von Polyolefin-Formteilen aufgebracht wird. Der in Anspruch 1 der EP-B-372 890 verwendete Ausdruck "mit der Faseroberfläche verhaftet" ist demnach vom Fachmann klar in der Weise zu verstehen, daß es sich dabei lediglich um eine lockere und temporäre Haftung - etwa durch relativ schwache Adhäsionskräfte - handelt, keinesfalls aber um eine dauerhafte Verankerung.

**EP-B-616 622** betrifft extrudierbare, kompostierbare Polymerzusammensetzungen, umfassend ein extrudierbares, thermoplastisches Polymer, Copolymer oder Mischungen davon, die ein abbauförderndes System aus einem autooxidativen Bestandteil und einem Übergangsmetall enthält. Das autooxidative System umfaßt dabei eine Fettsäure, eine substituierte Fettsäure oder Derivate oder Mischungen davon, wobei die Fettsäure 10 bis 22 C-Atome aufweist und mindestens 0,1 Gew.-% ungesättigter Verbindungen und mindestens 0,1 Gew.-% freie Säure enthält. Das Übergangsmetall ist in der Zusammensetzung in einer Menge von 5-500 ppm in Form eines Salzes enthalten und ausgewählt aus der Gruppe Kobalt, Mangan, Kupfer, Cer, Vanadium und Eisen. Die Zusammensetzung soll in Form einer Folie einer Dicke von etwa 100 Mikron bei 60 °C und einer relativen Feuchtigkeit von mindestens 80% innerhalb von 14 Tagen oxidativ zur Versprödung abbaubar sein.

**WO 98/42898** beschreibt die Verwendung von Amphiphilen zur dauerhaften Hydrophilisierung der Oberflächen von Polyolefin-basierten Formkörpern, Fasern und Folien. Dabei unterwirft man eine Mischung enthaltend (a) überwiegend ein oder mehrere Polyolefine, (b) 0,01 bis 10 Gew.-% - bezogen auf die Polyolefine - ein oder mehrerer migrationsfähiger Amphiphile und (c) 0,01 bis 1000 ppm ein oder mehrerer Übergangsmetall-Verbindungen - Metallgehalt der Übergangsmetall-Verbindungen bezogen auf die Polyolefine - bei Temperaturen im Bereich von 180 bis 320 °C auf übliche Weise einer formgebenden Verarbeitung wie Extrusions-, Kalandrier-, Spritzgußverfahren und dergleichen. Gemäß der technischen Lehre der WO 98/42898 ist bei der Herstellung der hydrophilisierten Polyolefin-Formkörper zwingend, daß alle drei Merkmale (a), (b) und (c) beachtet werden, d.h. daß bei der Herstellung zwingend eine Übergangsmetallverbindung, die als Katalysator anzusehen ist, eingesetzt wird.

Es ist auch bekannt, die Eigenschaften der Kunststoff-Oberfläche zur Erzielung spezieller Effekte durch beispielsweise oxidative Nachbehandlungsverfahren wie Corona- oder Plasmabehandlung zu verbessern. Hierbei wird der Kunststoff in Gegenwart von Gasen und Entladungen an der Oberfläche oxidiert oder chemisch modifiziert, wodurch sich gewisse Oberflächen-Eigenschaften des Kunststoffs modifizieren lassen. Diese Methoden erfordern jedoch neben einem hohen Energieeinsatz stets einen zusätzlichen Arbeitsgang und führen zu Ozonemissionen bei der Fertigung von Kunststoffteilen. Daneben sind chemische Vorbehandlungsverfahren wie z.B. das Behandeln mit Fluor- oder Chlorgas, mit Chromschwefelsäure oder Fluorsulfonsäure, usw. seit längerem bekannt.

### Beschreibung der Erfindung

Aufgabe der vorliegenden Erfindung war es, Arbeitsmittel bereitzustellen, mit denen Fasern auf Basis von Polyolefinen oder Polyester sowie davon abgeleitete Werkstoffe wie Vliese nachhaltig hydrophil ausgerüstet und der mehrfachen Benetzung durch wäßrige Medien - beispielsweise Urin - zugänglich gemacht werden können.

Der Begriff der Hydrophilisierung - in jüngerer Zeit spricht man auch oft von Hydrophilierung - ist dem Fachmann wohlvertraut. Hierzu wird in dem bekannten Standardwerk "**Lexikon für Textilveredlung**" (Herausgeber: Hans-Karl Rouette; Band 1; Dülmen 1995, Seiten 859-862) ausgeführt, daß durch Hydrophilisierung hydrophobe Fasern für Wasser benetzbar gemacht werden, was etwa für eine bessere Auswaschbarkeit von Synthesefaserartikeln wie auch für einen besseren Tragekomfort solcher Artikel sinnvoll ist. Beispiele für meßtechnische Indikatoren einer erfolgreichen Hydrophilisierung sind etwa das Netzen (die Oberflächenausbreitung einer Flüssigkeit) oder die Steighöhe (ein Maß für die Geschwindigkeit, mit der Wasser in textilen Flächengebilden entgegen der Schwerkraft transportiert wird).

Im Rahmen der vorliegenden Erfindung wird unter Hydrophilisierung - synonym hierfür wird im folgenden auch der Begriff der "hydrophilen Ausrüstung" verwendet - verstanden, daß Polyolefin- bzw. Polyester-Oberflächen, die mit Wasser einen Benetzungswinkel von mehr als 90° bilden - also "hydrophobe" Grenzflächen - durch eine spezielle Maßnahme so modifiziert werden, daß ihr Grenzwinkel nach dieser Maßnahme zu kleineren Werten hin verschoben ist. Es handelt sich hier um eher dynamische Wechselwirkungen entsprechend modifizierter Polymeroberflächen mit Molekülen oder Substraten, die nicht immobilisiert, sondern in flexibler Weise mit der Polymeroberfläche in Kontakt stehen. In dieser Hinsicht grenzen sich die Wirkungen, die unter den Begriff der Hydrophilisierung fallen, ausdrücklich von Phänomenen ab, bei denen Moleküle oder Substraten in dauerhafter Fixierung mit der Polymeroberfläche in Kontakt stehen, wie es etwa bei Beschichtungen und Verklebungen oder beim Färben und Bedrucken der Fall ist.

Gegenstand der Erfindung ist ein Verfahren zur hydrophilen Ausrüstung von Fasern, die ausschließlich oder ganz Polyolefine oder Polyester enthalten, wobei man eine Mischung enthaltend (a) überwiegend Polyolefine oder Polyester und (b) 0,01 bis 10 Gew.-% - bezogen auf die Summe von Polyolefin und Polyester - einer Zusammensetzung, die mindestens eine Verbindung aus der Klasse der Fettsäureamide enthält, bei Temperaturen im Bereich von 180 bis 320 °C auf übliche Weise einer formgebenden Verarbeitung zu Fasern unterwirft, wobei man
(i) der Mischung, die man der formgebenden Verarbeitung unterwirft, vor oder während dieser formgebenden Verarbeitung keine Übergangsmetallverbindungen zudosiert,
(ii) die Fettsäureamide (b) auswählt aus der Klasse der Fettsäurealkanolamide oder - dialkanolamide, wobei die diesen Verbindungen zu Grunde liegenden Fettsäuren gesättigte Fettsäuren mit 12-22 C-Atomen sind und daß
(iii) die Fettsäureamide (b) im individuellen Benetzungstest mindestens vier Cyclen bis zum Versagen benötigen.

Der "individuelle Benetzungstest" ist wie folgt durchzuführen:
1. Man vermengt 600 g eines hochmolekularen Polypropylen-Granulates (Handelsprodukt "Eltex PHY 671" der Firma Solvay) mit 9,0 g (=1,5 Gew.-%) der - hinsichtlich einer hydrophilen Ausrüstung - zu prüfenden Substanz. Diese Mischung wird durch einen Trichter in einen Extruder eingebracht (Doppelschneckenextruder DSK 42/7 der Firma Brabender OHG / Duisburg). Ein Extruder ist - wie dem Fachmann hinlänglich bekannt - eine Kunststoff-Verarbeitungsmaschine, welche zum kontinuierlichen Mischen und Plastifizieren sowohl von pulver- als auch granulatförmigen Thermoplasten geeignet ist. Unter dem Einfülltrichter befindet sich neben einer Wasserkühlung, die ein verfrühtes Schmelzen des Granulates bzw. Pulvers verhindern soll, auch eine gegenläufige Doppelschnecke, die der Länge nach in drei Heizzonen aufgeteilt ist. Die Temperatur der Heizzonen und die Drehzahl der Doppelschnecke lassen sich über einen Datenverarbeitungs-Plast-Corder PL 2000 regeln, der über eine PC-Schnittstelle mit dem Extruder verbunden ist. Dabei werden die Heizzonen I, II und III auf eine Temperatur von jeweils 200°C eingestellt, wobei die drei Heizzonen luftgekühlt werden, um die Temperatur konstant zu halten. Die Mischung von Polyopropylen-Granulat und Prüfsubstanz wird automatisch durch die gegeneinander laufende Doppelschnecke in den Extruder eingezogen und entlang der Schnecke befördert. Die Drehzahl wird auf 25 Umdrehungen pro Minute eingestellt, um eine gute Durchmischung und Homogenisierung zu gewährleisten. Diese homogene und praktisch bläschenfreie Mischung gelangt schließlich in eine Düse, die eine vierte Heizzone darstellt. Die Temperatur dieser Düse wird auf 200 °C eingestellt - bei dieser Temperatur verläßt also die Mischung den Extruder. Die Düse wird so gewählt, daß der mittlere Durchmesser des Stranges nach dem Austritt aus dieser Düse im Bereich von etwa 2 - 3 mm liegt. Dieser Strang wird granuliert, d.h. in kleine Stücke geschnitten, wobei man Längen von etwa 2-4 mm einstellt. Das erhaltene Granulat läßt man auf 20 °C abkülen. Dieses Granulat wird in einer Schmelzspinnanlage bei einer Verarbeitungstemperatur von 280 °C (d.h man stellt sowohl die Schmelzsterntemperatur als auch die Temperatur der Spinndüse auf 280 °C ein) gravimetrisch (d.h. durch Schwerkrafteinwirkung) in Fasern überführt. Die erhaltenen Fasern weisen einen Fasertiter im Bereich von etwa 10 - 30 dtex auf (1 dtex entspricht 1 g Faser pro 10000 m Faserlänge). Anschließend werden 500 m dieser Faser auf eine Rolle mit einem Durchmesser von 6,4 cm aufgewickelt. Diese auf die Rolle aufgewickelte Faser wird von der Rolle abgezogen und das abgezogene kreisförmige Gebilde durch mittiges Verknoten stabilisiert, wobei ein Gebilde erhalten wird, das die Form einer "8" hat; dieses Gebilde wird nachfolgend als "Strängchen" bezeichnet.
2. Man füllt einen 1-l-Meßzylinder (Glaszylinder mit einem Innendurchmesser von 6,0 cm) mit destilliertem Wasser von 20 °C und zwar bis zur 1000-ml-Markierung. Nun hält man das zu prüfende Strängchen in der Weise, das seine Längsrichtung mit der Vertikale des Meßzylinders übereinstimmt, d.h. daß das Strängchen als vertikale "8" erscheint. An den untersten Teil dieser "8" hängt man nun ein Gewicht, das aus Cu-Draht besteht, wobei die Masse des Cu-Drahtes 0,2064 g Cu pro Gramm Strängchen beträgt. Dieser Cu-Draht wird in Form von Windungen an dem Strängchen befestigt, wobei der Durchmesser der Cu-Draht-Windungen etwa 1 bis 2 cm beträgt; anschließend werden diese Cu-Draht-Windungen durch leichtes Drücken zwischen Daumen und Zeigefinger zusammengepreßt. Nun hält man das Strängchen mit dem Cu-Gewicht über die Wasseroberfläche des Meßzylinders und zwar so, daß der untere Teil des Cu-Gewichtes in das Wasser eintaucht und der unterste Teil des Strängchens sich etwa 2 mm über der Wasseroberfläche befindet Dann läßt man das Strängchen los und mißt mit einer Stoppuhr die Zeit in Sekunden, die das Strängchen benötigt, um von der 1000-ml-Markierung bis zur 200-ml-Markierung zu gelangen. Beginn und Ende der Meßzeit sind dadurch definiert, daß das unterste Ende des Strängchens jeweils die 1000-ml- bzw. die 200-ml-Marke passiert. In der vorliegenden Versuchsanordnung bedeutet das, daß das Strängchen auf seinem Weg zwischen den genannten Markierungen eine Tauchstrecke von 28,3 cm zurückgelegt hat. Dieser erste Meßwert wird als C1-Wert ("Wert des ersten Benetzungscyclus") bezeichnet.
3. Das Strängchen wird unmittelbar nach Bestimmung des C1-Wertes aus dem Meßzylinder genommen, mit Zellstoff abgetupft und 1 Stunde in einem Umlufttrockenschrank (Typ UT 5042 EK der Firma Heraeus) bei 40 °C getrocknet. Anschließend wird Schritt 2 wiederholt. Der jetzt erhaltene Wert in Sekunden der 28,3-cm-Sinkzeit wird als C2-Wert ("Wert des zweiten Benetzungscyclus") bezeichnet. Trocknung und Bestimmung der 28,3-cm-Sinkzeit werden nun erneut wiederholt, wobei man den C3-Wert ("Wert des dritten Benetzungscyclus") erhält. Trocknung und Bestimmung der 28,3-cm-Sinkzeit werden nun erneut wiederholt, wobei man den C4-Wert ("Wert des vierten Benetzungscyclus") erhält. Trocknung und Bestimmung der 28,3-cm-Sinkzeit werden nun erneut wiederholt, wobei man den C5-Wert ("Wert des fünften Benetzungscyclus") erhält. Gewünschtenfalls kann die beschriebene Prozedur auch noch mehrfach wiederholt werden, es können also bei Bedarf auch noch C5-, C6-, C7- usw. Werte bestimmt werden.
4. Sofern 28,3-cm-Sinkzeiten (Cl- bis C5-Werte) oberhalb von 180 Sekunden liegen, wird der jeweilige Cyclus beendet.
5. Sofern an dem Strängchen, vorwiegend in dessen oberen Teil, beim Eintauchen in das Wasser des Meßzylinders Luftblasen hängenbleiben, werden diese sofort mittels eines Drahtes oder durch kurzes Berühren mit einem Finger entfernt.

Der individuelle Benetzungstest gilt als bestanden, wenn die Werte für C1, C2 und C3 jeweils maximal 50 Sekunden oder weniger betragen. Dies ist gleichbedeutend damit, daß beim Einsatz einer erfindungsgemäß geeigneten Prüfsubstanz, d.h eines Fettsäureamids (b), zur hydrophilen Ausrüstung des Polypropylens frühestens beim Durchlaufen des vierten Cyclus (C4-Wert) ein Wert oberhalb von 50 Sekunden erreicht wird. Ein Fettsäureamid (b) ist dann geeignet im Sinne der vorliegenden Erfindung, wenn ein Versagen, also ein 28,3-cm-Sinkzeiten-Wert oberhalb von 50 Sekunden, frühestens beim C4-Wert eintritt.

Die erfindungsgemäß einzusetzenden Fettsäureamide (b) sind strukturell - wie bereits gesagt - der Klasse der Fettsäuremonoalkanolamide bzw. Fettsäuredialkanolamide zuzuordnen, wobei die diesen Verbindungen zu Grunde liegenden Fettsäuren gesättigte Fettsäuren mit 12-22 C-Atomen sind. Diese Verbindungen können sowohl einzeln als auch in Kombination miteinander eingesetzt werden.

In einer bevorzugten Ausführungsform gilt die zusätzliche Maßgabe, daß die Mischung enthaltend die Komponenten a) und b) Fettsäureamide auf Basis ungesättigter Fettsäuren höchstens in einer Menge enthält, die 40 Gew.-% - bezogen auf die Menge der Fettsäureamide b) auf Basis gesättigter Fettsäuren - entspricht. Vorzugsweise gilt die zusätzliche Maßgabe, daß die Mischung enthaltend die Komponenten a) und b) Fettsäureamide auf Basis ungesättigter Fettsäuren höchstens in einer Menge enthält, die 10 Gew.-% - bezogen auf die Menge der Fettsäureamide b) auf Basis gesättigter Fettsäuren - entspricht

In einer bevorzugten Ausführungsform setzt man solche Fettsäuremonoalkanolamide bzw. Fettsäuredialkanolamide ein, deren zu Grunde liegende Fettsäuren gesättigte Fettsäuren mit 12-16 und insbesondere 12-14 C-Atomen sind. Besonders bevorzugte Verbindungen dieser Klasse sind die Mono- und Di-Ethanolamide von Laurinsäure, Myristinsäure sowie in pflanzlichen Rohstoffen vorkommenden Gemischen dieser Säuren, beispielsweise Kokosfettsäure. Die genannten Verbindungen werden insbesondere in technischer Qualität eingesetzt.

In einer besonders bevorzugten Ausführungsform der Erfindung setzt man solche Verbindungen (b) ein, die im individuellen Benetzungstest mindestens sechs Cyclen bis zum Versagen benötigen. Bei diesen Verbindungen sind also sämtliche Werte C1, C2, C3, C4 und C5 im oben beschriebenen individuellen Benetzungstest unterhalb von 50 Sekunden.

Die erfindungsgemäß einzusetzenden Verbindungen (b) sind zur Migration befähigt. Darunter ist zu verstehen, daß diese Verbindungen in der Lage sind, im Zuge der Herstellung der Fasern durch beispielsweise Extrusionsverfahren an die Oberfläche des resultierenden Polyolefin- bzw. Polyester-Formkörpers zu gelangen. Sie reichern sich dadurch an der Oberfläche bzw. den Oberflächen-nahen Bereichen der Kunststoff-Matrix an, was durch sukzessives Abtragen von Oberflächenschichten in der Größenordnung von jeweils wenigen Nanometern und anschließende Abscan-Techniken von der Anmelderin verifiziert wurde.

Die durch das erfindungsgemäße Verfahren zugänglichen Polyolefin- bzw. Polyesterfasern sowie daraus herstellbare textile Flächengebilde - etwa Vliese - zeichnen sich durch eine ausgezeichnete Benetzbarkeit durch wäßrige Medien aus.

Durch das technische Handeln im Sinne der erfindungsgemäßen Lehre wird einerseits sichergestellt, daß die angestrebte verbesserte und dauerhafte Oberflächen-Hydrophilie erreicht wird, andererseits, daß dies ohne Beeinträchtigung anderer Werkstoffparameter gelingt.

Die Einarbeitung der Fettsäureamide (b) in die Kunststoff-Matrix geschieht im Rahmen üblicher formgebender Verarbeitungsprozesse wie Extrusionsverfahren und dergleichen. Dabei kann es gewünscht sein, eine vorkonfektionierte Mischung der Komponenten a) und b) einzusetzen. Mitverwendete weitere übliche Hilfsstoffe, die sich bei der Verarbeitung von Kunststoffen allgemein bewährt haben und die dem Fachmann bekannt sind, beispielsweise Slipmittel, Antistatika, Gleitmittel, Trennmittel, UV-Stabilisatoren, Antioxidantien, Füllstoffe, Brandschutzmittel, Entformungsmittel, Nukleirungsmittel und Antiblockmittel können entsprechend in getrennter Form vorkonfektioniert und bei der abschließenden Aufmischung der Fertigprodukte zugegeben werden. Es kann aber - beispielsweise bei Anwendung der Extrudiertechnik - auch gewünscht sein, die Komponenten b) und/ oder andere Additive ganz oder teilweise direkt in die Polymerschmelze am Extruder einzudosieren, so daß die Mischung der Komponenten a) und b) - und gegebenenfalls weiterer Hilfsstoffe - nicht schon von vornherein als Vorkonfektionat vorhanden ist, sondern erst im Extruder selbst vorliegt. Eine derartige Technik bietet sich beispielsweise dann an, wenn die der Polymerschmelze zuzudosierenden Verbindungen b) in flüssiger Form vorliegen und ein Einspritzen dieser Komponente einfacher ist, als eine Vorkonfektionierung.

Es kann auch gewünscht sein - obgleich zur Erzielung des erfindungsgemäßen Effektes nicht erforderlich - im Anschluß an den erfindungsgemäßen Einsatz der Komponenten a) und b) auf übliche Weise eine Corona- oder Plasmabehandlung vorzunehmen.

Als Komponente a) sind im Rahmen der Erfindung Polyolefine bevorzugt. Hier eigenen sich an sich alle heute bekannten Polymer- und Copolymertypen auf Ethylen- beziehungsweise Propylen-Basis. Auch Abmischungen reiner Polyolefine mit Copolymeren sind grundsätzlich geeignet. Für die erfindungsgemäße Lehre besonders geeignete Polymertypen sind in der nachfolgenden Zusammenstellung aufgezählt:

Poly(ethylene) wie HDPE (high density polyethylene), LDPE (low density polyethylene), VLDPE (very low density polyethylene), LLDPE (linear low density polyethylene), MDPE (medium density polyethylene), UHMPE (ultra high molecular polyethylene), VPE (vernetztes Polyethylen), HPPE (high pressure polyethylene); isotaktisches Polypropylen; syndiotaktisches Polypropylen; Metallocen-katalysiert hergestelltes Polypropylen, schlagzäh-modifiziertes Polypropylen, Random-Copolymere auf Basis Ethylen und Propylen, Blockcopolymere auf Basis Ethylen und Propylen; EPM (Poly[ethylen-copropylen]); EPDM (Poly[ethylen-co-propylen-co-konjugiertes Dien]).

Weitere geeignete Polymertypen sind: Poly(styrol); Poly(methylstyrol); Poly(oxymethylen); Metallocen-katalysierte alpha-Olefin- oder Cycloolefin-Copolymere wie Norbornen-Ethylen-Copolymere; Copolymere, die zu mindestens 80 % Ethylen und/oder Styrol enthalten und zu weniger als 20 % Monomere wie Vinylacetat, Acrylsäureester, Methacrylsäureester, Acrylsäure, Acrylnitril, Vinylchlorid. Beispiele solcher Polymeren sind: Poly(ethylen-co-ethylacrylat), Poly(ethylen-co-vinylacetat), Poly(ethylen-co-vinylchlorid), Poly(styrol-co-acrylnitril). Geeignet sind weiterhin Pfropfcopolymere sowie Polymerblends, das heißt, Mischungen von Polymeren, in denen unter anderem die vorgenannten Polymere enthalten sind, beispielsweise Polymerblends auf Basis von Polyethylen und Polypropylen.

Im Rahmen der vorliegenden Erfindung sind Homo- und Copolymere auf Basis von Ethylen und Propylen besonders bevorzugt. In einer Ausführungsform der vorliegenden Erfindung setzt man dementsprechen als Polyolefin ausschließlich Polyethylen ein, in einer anderen Ausführungsform ausschließlich Polypropylen, in einer weiteren Ausführungsfrom Copolymere auf Basis von Ethylen und Propylen.

In einer ganz besonders bevorzugten Ausführungsform der Erfindung ist Komponente a) Polypropylen.

Ein weiterer Gegenstand der Erfindung ist die Verwendung der gemäß dem oben beschriebenen Verfahren hergestellten hydrophilisierten und durch wäßrige Medien benetzbaren Fasern auf Polyolefin bzw. Polyester-Basis zur Herstellung textiler Flächengebilde. Vorzugsweise sind dabei die textilen Flächengebilde Vliesstoffe. In einer besonders bevorzugten Ausführungsform sind diese textilen Flächengebilde zum Einsatz in Windeln bestimmt.

Für den letztgenannten Fall, den Einsatz von textilen Flächengebilden in Windeln, stellt der individuelle Benetzungstest eine geeignete Simulation dar. Windeln werden nämlich üblicherweise über einen Zeitraum von 3 bis 5 Stunden getragen, wobei ihre Innenseite durchschnittlich bis zu 3-mal mit Urin benetzt wird. Es muß dann gewährleistet sein, daß ein hydrophil ausgerüstetes Vlies auf Basis eines ansonsten hydrophoben Kunststoffs jeweils ausreichend benetzbar ist, so daß der Urin durch das Vlies penetrieren und vom Absorbermaterial der Windel gebunden werden kann.

Vliesstoffe können nach allen im Stand der Technik bekannten Verfahren der Vliesherstellung, wie sie beispielsweise in **Ullmann's Encyclopedia of Industrial Chemistry**, Vol. A 17, VCH Weinheim 1994, Seiten 572 - 581, beschrieben werden, hergestellt werden. Bevorzugt sind dabei Vliese, die entweder nach dem sogenannte "dry laid"- oder dem Spinnvlies- oder spunbond-Verfahren hergestellt wurden. Das "dry laid"-Verfahren geht von Stapelfasern aus, die üblicherweise durch Kardieren in Einzelfasern getrennt und anschließend unter Einsatz eines aerodynamischen oder hydrodynamischen Verfahrens zum unverfestigten Vliesstoff zusammengelegt werden. Dieser wird dann beispielsweise durch eine thermische Behandlung zum fertigen Vlies verbunden (das sogenannte "thermobonding"). Dabei werden die synthetischen Fasern entweder soweit erwärmt, daß deren Oberfläche schmilzt und die Einzelfasern an den Kontakstellen miteinander verbunden werden, oder die Fasern werden mit einem Additiv überzogen, welches bei der Wärmebehandlung schmilzt und so die einzelnen Fasern miteinander verbindet. Durch Abkühlung wird die Verbindung fixiert. Neben diesem Verfahren sind natürlich auch alle anderen Verfahren geeignet, die im Stand der Technik zum Verbinden von Vliesstoffen eingesetzt werden. Die Spinnvliesbildung geht dagegen von einzelnen Filamenten aus, die nach dem Schmelzspinnverfahren aus extrudierten Polymeren gebildet werden, welche unter hohem Druck durch Spinndüsen gedrückt werden. Die aus den Spinndüsen austretenden Filamente werden gebündelt, gestreckt und zu einem Vlies abgelegt, welches üblicherweise durch "thermobonding" verfestigt wird.

### Beispiele

Mit unterschiedlichen Prüfsubstanzen (B1 bis B3 = erfindungsgemäße Beispiele; V1 bis V4 = Vergleichsversuche) ausgerüstete Polypropylen-Prüfkörper wurden dem oben beschriebenen individuellen Benetzungstest unterworfen. Die Versuchsergebnisse sind in Tabelle 1 zusammengestellt; angegeben sind dabei die 28,3-cm-Sinkzeiten (in Sekunden) nach ein, zwei, drei, vier und fünf Benetzungscyclen (Spalten C1 bis C5).

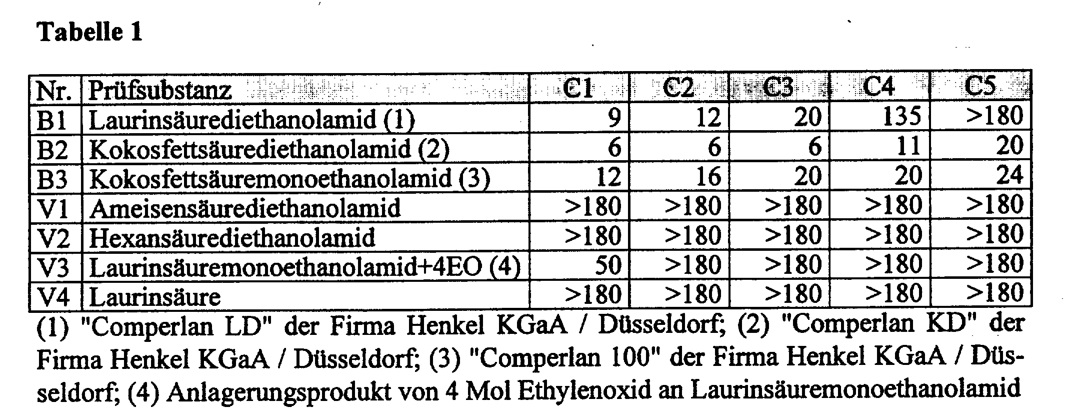

Es ist deutlich, daß die Werte C1, C2 und C3 bei den Beispielen B1 bis B3 sämtlich unterhalb des kritischen Wertes von 50 Sekunden liegen und den entsprechenden Werten der Vergleichsbeispiele V1 bis V4 deutlich überlegen sind. Die Beispiele B2 und B3 zeigen sogar noch nach fünf Benetzungscyclen ganz ausgezeichnete Werte.

## Patentansprüche

1. Verfahren zur hydrophilen Ausrüstung von Fasern, die ausschließlich oder ganz Polyolefine oder Polyester enthalten, wobei man eine Mischung enthaltend (a) überwiegend Polyolefine oder Polyester und (b) 0,01 bis 10 Gew.-% - bezogen auf die Summe von Polyolefin und Polyester - einer Zusammensetzung, die mindestens eine Verbindung aus der Klasse der Fettsäureamide enthält, bei Temperaturen im Bereich von 180 bis 320 °C auf übliche Weise einer formgebenden Verarbeitung zu Fasern unterwirft, dadurch gekennzeichnet, daß man
(i) der Mischung, die man der formgebenden Verarbeitung unterwirft, vor oder während dieser formgebenden Verarbeitung keine Übergangsmetallverbindungen zudosiert,
(ii) die Fettsäureamide (b) auswählt aus der Klasse der Fettsäurealkanolamide oder -dialkanolamide, wobei die diesen Verbindungen zu Grunde liegenden Fettsäuren gesättigte Fettsäuren mit 12-22 C-Atomen sind und daß
(iii) die Fettsäureamide (b) im individuellen Benetzungstest mindestens vier Cyclen bis zum Versagen benötigen.

2. Verfahren nach Anspruch 1, wobei man als Komponente a) Polypropylen einsetzt.

3. Verfahren nach Anspruch 1 oder 2, wobei man als Komponente b) Mono- und/oder Diethanolamide von Laurinsäure und/oder Myristinsäure einsetzt.

4. Verfahren nach einem der Ansprüche 1 bis 3, wobei man bei der formgebenden Verarbeitung übliche weitere Hilfsstoffe zur Verarbeitung von Kunststoffen zusetzt und/oder als zusätzlichen weiteren Verarbeitungsschritt auf übliche Weise eine Corona- oder Plasmabehandlung vornimmt.

5. Verfahren um nichtgewebten Materialien, die Fasern aus Polyolefin oder Polyester enthalten, hydrophile Eigenschaften zu verleihen und sie für wäßrige Medien durchlässig zu machen, dadurch gekennzeichnet, daß man die gemäß dem Verfahren nach einem der Ansprüche 1 bis 4 hergestellten hydrophil ausgerüsteten Fasern auf Basis von Polyolefin oder Polyester auf übliche Weise - beispielsweise zu Vliesstoffen - weiterverarbeitet.

6. Verwendung der gemäß dem Verfahren nach einem der Ansprüche 1 bis 4 hergestellten hydrophilisierten und durch wäßrige Medien benetzbaren Fasern auf Polyolefin- oder Polyester-Basis zur Herstellung textiler Flächengebilde.

7. Verwendung nach Anspruch 6, wobei die textilen Flächengebilde Vliesstoffe sind.

8. Verwendung nach Anspruch 6 oder 7, wobei die textilen Flächengebilde zum Einsatz in Windeln bestimmt sind.
